# EUROPEAN PATENT SPECIFICATION

(11) **EP 2 869 798 B1**
(45) Date of publication and mention of the grant of the patent: **01.09.2021**
(21) Application number: 13812993.7
(22) Date of filing: 01.07.2013
(51) Int. Cl.: A61F 5/44, A61F 5/445

(54) **OSTOMY DEVICES**
Ostomievorrichtungen
DISPOSITIFS DE STOMIE

(30) Priority: 03.07.2012 US 201261667762 P
(43) Date of publication of application: 13.05.2015
(73) Proprietor: MAYO FOUNDATION FOR MEDICAL EDUCATION AND RESEARCH, Rochester, MN 55905 (US)
(72) Inventor: MAIDL, Laurie J., Rochester, Minnesota 55901 (US); FAUBION, William A., Rochester, Minnesota 55902 (US); DOZOIS, Eric J., Rochester, Minnesota 55905 (US); ZIEGLER, Troy J., Apple Valley, Minnesota 55124 (US)
(74) Representative: Grund, Martin
(86) International application number: PCT/US2013/048953
(87) International publication number: WO 2014/008198

(56) References cited:
- EP-A1- 2 407 129
- WO-A1-03/065946
- US-A- 4 338 937
- US-A- 4 351 322
- US-A1- 2009 157 140
- US-A1- 2011 060 362
- US-A1- 2012 116 447
- None

## Description

### BACKGROUND

### 1. Technical Field

This document relates to ostomy devices. For example, this document relates to plug devices for an ostomy (e.g., a colostomy, ileostomy, or urostomy).

### 2. Background Information

Treating some diseases of the digestive or urinary systems can involve removing all or part of a patient's small intestine, colon, rectum, or bladder. In these cases, waste must be rerouted to exit the body of the subject. The rerouting surgery, known as an ostomy, can involve creating an opening in the abdomen wall so that a portion of the intestinal tract can be brought out to the skin level, resulting in what is called a stoma. Three common types of abdominal wall stomas result from, and may be classified as, a colostomy, ileostomy, and urostomy, which involve patients who have had surgery on their the large intestine, small intestine, and urinary bladder, respectively. Typically, a medical prosthetic known as an ostomy pouching system can be used to collect waste from a diverted biological system as it exits a stoma. The patent US 4351322A discloses a stoma device which is designed to provide control over the discharge from a person's stoma. This stoma device comprises an ostomy plug device for sealing a stoma, wherein said plug device comprises a sealing element, a cap element, and a tensioning element. The tensioning element of US4351322A consists of a balloon and is not attached to the cap element.

### SUMMARY

The scope of the invention is determined by the appended claims. This document provides materials related to ostomy devices. This document provides plug devices for an ostomy (e.g., a colostomy, ileostomy, or urostomy).

As described herein, an ostomy plug device is configured to include a sealing element, a tensioning element, and a cap element. The sealing element is positioned within a waste collection reservoir of an ostomy patient. The tensioning element is connected to the sealing element and the cap element in a manner that allows the tensioning element to provide sufficient tension on the sealing element. In some cases, the tension applied to the sealing element from the tensioning element can be sufficient to prevent waste material and/or gas from exiting a waste collection reservoir, while applying minimal pressure against a stricture. The cap element is configured to be located outside of the ostomy patient. Such cap elements can be configured to cover a stoma and are configured to provide an anchoring structure for a tensioning element such that the tensioning element can provide sufficient tension to the sealing element. In some cases, a tensioning element of an ostomy plug device provided herein can be a solid cord or string and can lack a fluid or gas conduit. In some cases, an ostomy plug device provided herein can be configured to lack fluid and gas conduits that extend from outside a patient to a sealing element located within the patient. In some cases, when the sealing element is inflatable, an ostomy plug device can be configured to include one or more fluid and/or gas conduits capable of being used to inflate the inflatable sealing element. For example, a tensioning element can be in the form of a conduit capable of being used to inflate the inflatable sealing element.

In general, one aspect of this document features an ostomy plug device for sealing a stoma from within an ostomy patient. The ostomy plug device comprises, or consists essentially of, a sealing element, a cap element, and a tensioning element attached to the sealing element and the cap element, wherein the cap element is configured to be located outside of the body of the patient, wherein the sealing element is configured to be implanted through the stoma into at least a portion of a waste collection reservoir located within the patient, and wherein the tensioning element is configured to provide tension to the sealing element using the cap element for anchoring, thereby sealing the stoma from within an ostomy patient. The sealing element can be disc shaped. The sealing element can be biased to have a concave surface facing the stoma, wherein the tension applied to the sealing element by the tensioning element can cause the concave surface to evert to a convex surface. The sealing element can flex upon application of the tension. The cap element can define a port. The port can be configured to allow gas, waste material, or both to be removed from the waste collection reservoir. The cap element can be rigid. The tensioning element can be a cord or string. The tensioning element can be a flexible cord or string. The tensioning element can be solid and can lack a lumen. The waste collection reservoir can be a surgically created waste collection reservoir.

Unless otherwise defined, all technical and scientific terms used herein have the same meaning as commonly understood by one of ordinary skill in the art to which this invention pertains. Although materials similar or equivalent to those described herein can be used in the practice or testing of the present invention, suitable materials are described below. In case of conflict with references in this document, the specification of the present document, including definitions, will control. In addition, the materials and examples are illustrative only and not intended to be limiting.

Other features and advantages of the invention will be apparent from the following detailed description, and from the claims.

### DESCRIPTION OF DRAWINGS

Figure 1 is a side view of an ostomy plug device positioned within respect of an ostomy patient, in accordance with one embodiment provided herein.
Figures 2A and 2B are front and side views, respectively, of a sealing element of an ostomy plug device, in accordance with one embodiment provided herein.
Figures 2C and 2D are front and side views, respectively, of a sealing element of an ostomy plug device, in accordance with one embodiment provided herein.
Figures 2E and 2F are front and side views, respectively, of a sealing element of an ostomy plug device, in accordance with one embodiment provided herein.
Figures 2G and 2H are front and side views, respectively, of a sealing element of an ostomy plug device, in accordance with one embodiment provided herein.
Figures 3A is an elevated side view of a cap element of an ostomy plug device, in accordance with one embodiment provided herein.
Figures 3B is an elevated side view of a cap element of an ostomy plug device, in accordance with one embodiment provided herein.
Figure 4 is a side view of an ostomy plug device positioned within respect of an ostomy patient, in accordance with one embodiment provided herein.

### DETAILED DESCRIPTION

This document provides materials related to ostomy devices. For example, this document provides materials that can be used to prevent waste material and/or gas from escaping a waste reservoir of an ostomy patient.

In general, an ostomy plug device provided herein is configured to include a sealing element, a tensioning element, and a cap element. A sealing element is configured to be positioned within a waste collection reservoir of an ostomy patient, and a tensioning element is connected to the sealing element and a cap element in a manner that allows the tensioning element to provide sufficient tension on the sealing element so as to prevent waste material and/or gas from escaping a waste reservoir of an ostomy patient. In some cases, the tension applied to the sealing element from the tensioning element can be sufficient to prevent waste material and/or gas from exiting a waste collection reservoir, while applying minimal pressure against a stricture. The cap element is configured to be located outside of the ostomy patient. In some cases, a cap element can be configured to cover a stoma and is configured to provide an anchoring structure for a tensioning element such that the tensioning element can provide sufficient tension to the sealing element.

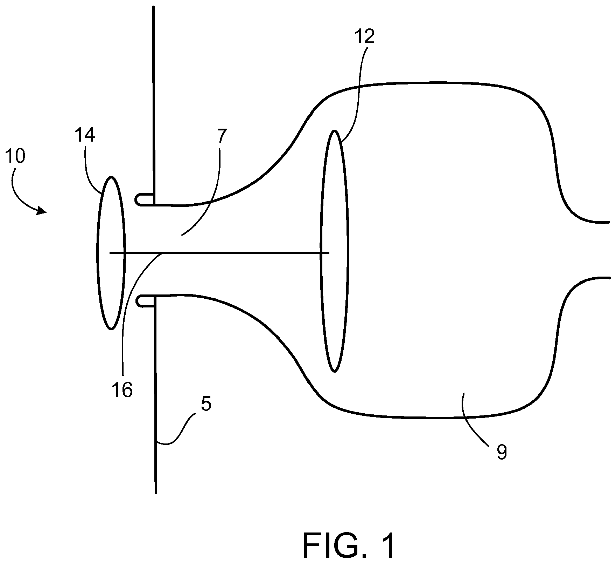

With reference to Figure 1, an ostomy plug device 10 can include a sealing element 12, a tensioning element 16, and a cap element 14. Tensioning element 16 can be attached to sealing element 12 and cap element 14. In some cases, tensioning element 16 can extend through cap element 14 such that a user can manually adjust the tension applied by tensioning element 16 on sealing element 12. For example, tensioning element 16 can be a cord, string, wire, or conduit that extend from sealing element 12 through cap element 14 such that a user can manually pull on one end of tensioning element 16 to adjust the tension applied by tensioning element 16 on sealing element 12. In some cases, tensioning element 16 can be configured to be flexible such that a predetermined amount of tension is applied to sealing element 12 based on the elasticity of the material of tensioning element 16 and the distance between cap element 14 and sealing element 12.

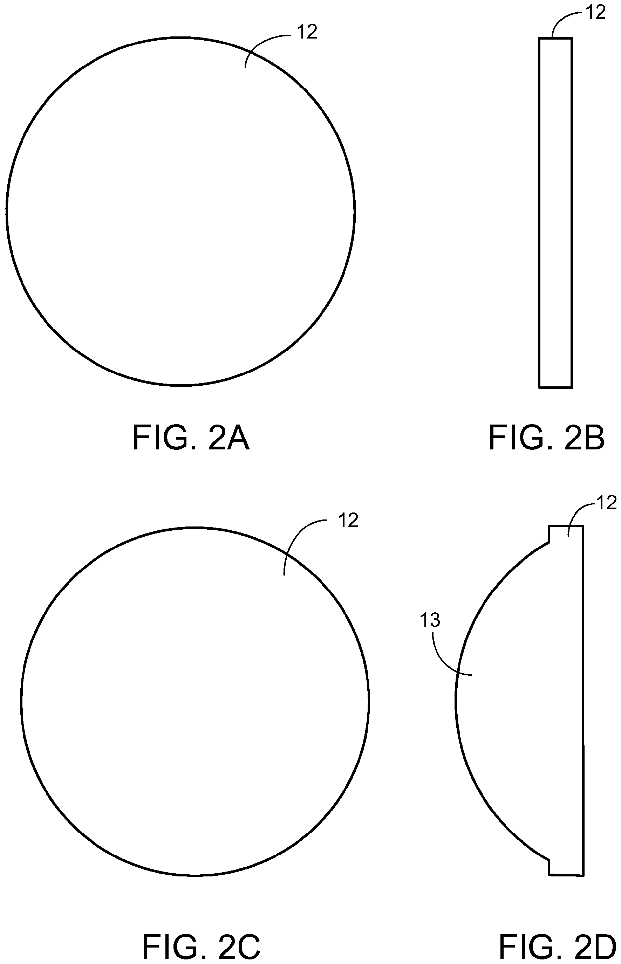

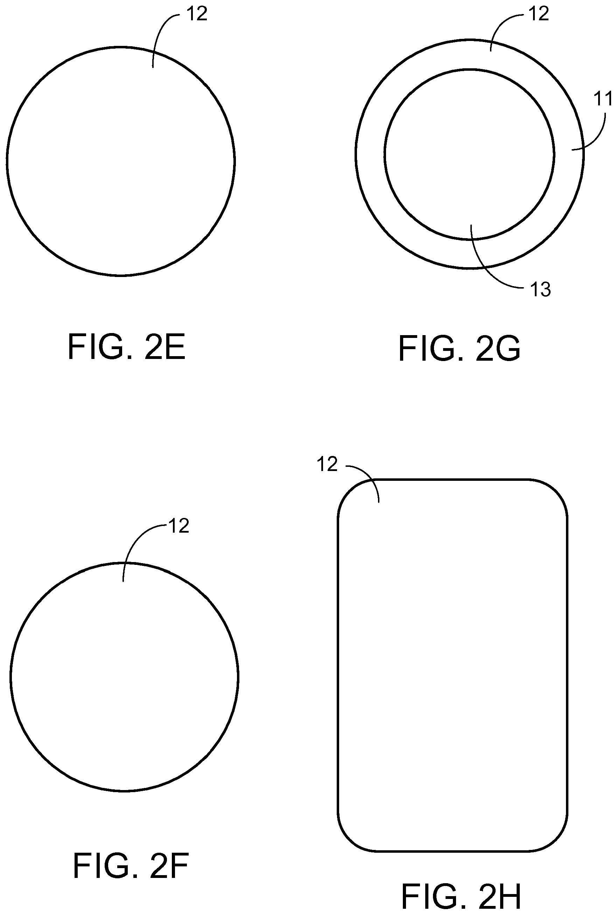

Sealing element 12 can have any appropriate shape and configuration provided that it is capable of preventing waste material and/or gas from exiting a waste collection reservoir 9 (e.g., a surgically created waste collection reservoir) when tension is applied by tensioning element 16. For example, sealing element 12 can be disk shaped as shown in Figures 2A and 2B, dome shaped as shown in Figures 2C and 2D, ball shaped as shown in Figures 2E and 2F, or covered ring shaped (e.g., a donut shape without a hole) as shown in Figures 2G and 2H. Other examples of shapes and configurations for a sealing element include, without limitation, bulbous lens, discs, saucers, and caps. As shown in Figure 2D, a dome shaped sealing element 12 can have one surface 13 that is convex and an opposing surface that is concave or flat with respect to that convex surface. Such a convex surface can be configured to extend into the opening of an ostomy in a manner that helps prevent waste material and/or gas from exiting the waste collection reservoir. As shown in Figure 2G, a covered ring shaped sealing element 12 can have an outer ring portion 11 and an inner portion 13.

In some cases, sealing element 12 can be inflatable (e.g., gel, fluid, or gas inflatable). For example, a ball shaped sealing element 12 as shown in Figures 2E and 2F or a covered ring shaped (e.g., a donut shape without a hole) sealing element 12 as shown in Figures 2G and 2H can be inflatable. In such cases, a conduit can extend from sealing element 12 to the outside of an ostomy patient such that sealing element 12 can be inflated and deflated as needed. In some cases, tensioning element 16 can be configured as a conduit for inflating and deflating an inflatable sealing element.

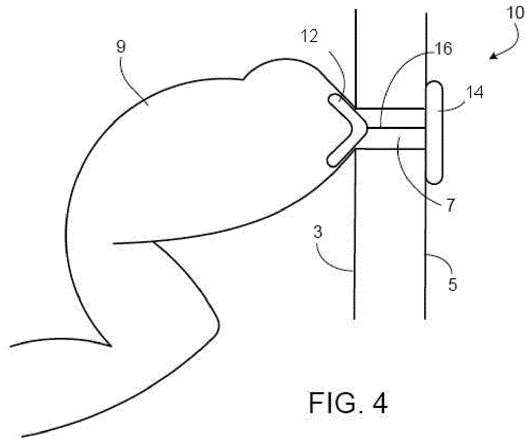

Sealing element 12 can be flexible. For example, a disk shaped sealing element 12 as shown in Figures 2A and 2B can be flexible such that when tension is applied by tensioning element the disk shaped sealing element 12 flexes in a direction as shown, for example, in Figure 4. Release of the tension by tensioning element 16 can allow the disk shaped sealing element 12 to return to its normal disk configuration. In some cases, the application of tension can be used when a user intends to prevent waste material and/or gas from exiting the waste collection reservoir, and the removal of tension can be used when the user intends to remove waste material and/or gas from the waste collection reservoir. In some cases, a user can completely remove sealing element 12 to remove waste material and/or gas from the waste collection reservoir.

In some cases, sealing element 12 can be a convex/concave everting structure. For example, sealing element 12 can be biased to have a concave surface facing the opening of an ostomy when sealing element 12 is under little or no tension from tensioning element 16. The application of tension from tensioning element 16 can cause sealing element 12 to evert such that the concave surface becomes a convex surface and forms a seal with an inner lining of the ostomy patient's waste collection reservoir. In such cases, release of at least some of the tension applied to sealing element 12 can allow sealing element 12 to evert back to its naturally biased configuration such that the surface facing the opening of the ostomy is a concave surface and the seal between sealing element 12 and an inner lining of the ostomy patient's waste collection reservoir is opened, thereby allowing waste material and/or gas to be removed from the waste collection reservoir.

Sealing element 12 can be made of any appropriate material. For example, sealing element 12 can be made of a biocompatible material such as a plastic, a polymer of a natural material, or a polymer of a synthetic material. In some cases, sealing element 12 can be soft and compliant in a manner that allows it to be easily inserted through a stricture 7 and can be rigid enough to prevent it from collapsing and/or breaking its seal. Sealing element 12 can have a large enough surface area to create an effective seal with minimal pressure against stricture 7. In some cases, a surface of sealing element 12 (e.g., a surface configured to engage an inner surface of the waste collection reservoir) can include tacky or sticky material. Such material can be in the form of a coating.

In some cases, a catheter, rod, or other device (e.g., a fenestrated obturator) can be advanced within a stoma to release a seal formed between sealing element 12 and an inner lining of the ostomy patient's waste collection reservoir. For example, to release a seal formed between sealing element 12 and an inner lining of the ostomy patient's waste collection reservoir, the tension applied to sealing element 12 by tensioning element 16 can be release and a catheter can be advanced into the stoma so as to push sealing element 12 away from the inner lining of the ostomy patient's waste collection reservoir. In such cases, the catheter can be used to remove waste material and/or gas from the waste collection reservoir.

Cap element 14 can have any appropriate shape and configuration provided that it is capable of providing an anchor for tensioning element 16. For example, cap element 14 can be disk shaped, dome shaped, covered ring shaped (e.g., a donut shape without a hole), or donut shaped. Other examples of shapes and configurations for a cap element include, without limitation, bulbous lens, discs, saucers, and caps. Cap element 14 can lack one or more holes or openings or can define one or more holes or openings. For example, cap element 14 can define an opening or port and can be configured to have a closing structure (e.g., a lid or plug) that can be used to close that opening or port to prevent access to the stoma or waste collection reservoir. Such closing structure can be opened or removed to provide access to the stoma or waste collection reservoir. In some cases, cap element 14 can have a valve that can be

actuated from a closed position to an open position to provide access to the stoma or waste collection reservoir.

In some cases, cap element 14 can cover the stoma to absorb and/or prevent leakage of mucus or other material from the stoma. In some cases, cap element 14 can include an adhesive material. Such adhesive material can be in the form of a coating and can help prevent movement of cap element 14. Examples of adhesive materials include, without limitation, reactive adhesives of natural origin, reactive adhesives of natural synthetic origin, non-reactive adhesives of natural origin, and non-reactive adhesives of synthetic origin.

In some cases, cap element 14 can include a tension indicator capable of providing a user with an indication of the degree of tension being applied by tensioning element 16 to sealing element 12. In some cases, cap element 14 can include a gas valve capable of being opened to release gas from the stoma and/or waste collection reservoir.

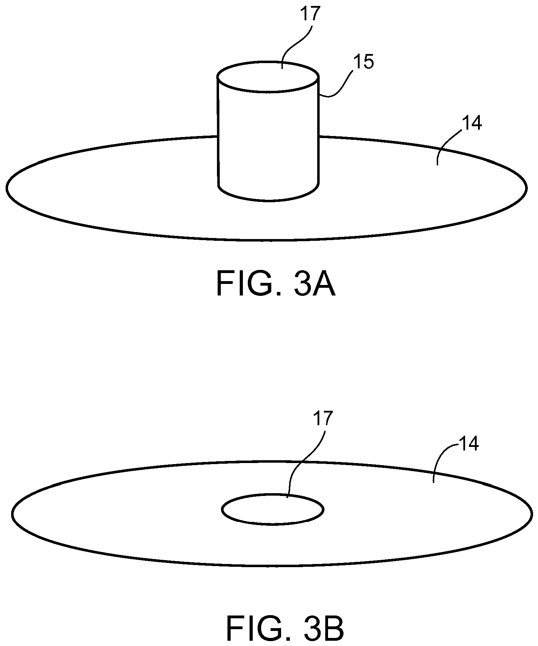

As shown in Figure 3A, cap element 14 can have a disk shaped base portion and an extended port 15 defining an opening 17. In some cases, extended port 15 can be capable of telescoping away from the base portion. In some cases, cap element 14 can define an opening 17 as shown in Figure 3B. In some cases, opening 17 can be a port (e.g., flanged port) that allows a device (e.g., an endoscope, camera, or catheter) to be inserted through the port while allowing little or no air to escape.

Cap element 14 can be rigid. For example, a disk shaped cap element 14 as shown in Figures 3A or 3B can be rigid such that cap element 14 provide an anchoring role when tension is applied sealing element 12 by tensioning element 16.

Cap element 14 can be made of any appropriate material. For example, cap element 14 can be made of a biocompatible material such as a plastic, a polymer of a natural material, or a polymer of a synthetic material.

In some cases, cap element 14 can work in conjunction with sealing element 12 to prevent waste material and/or gas from exiting a waste collection reservoir 9 when tension is applied by tensioning element 16.

With reference to Figure 4, ostomy plug device 10 can be inserted into an ostomy patient having a surgically created waste collection reservoir 9. Surgically created waste collection reservoir 9 can be formed by surgically combining intestinal material in a manner that results in a reservoir that has a larger diameter and larger volume than a similar length of intestine would naturally have. A stoma provides an exit point for waste material from a waste collection reservoir and can be in the form of a stricture 7. Stricture 7 can be composed of fascia 3 and/or fat and can extend to skin 5. In some cases, stricture 7 can define a stoma surgically created to have a smaller diameter than surgically created waste collection reservoir 9. In some cases, stricture 7 can formed by surgically inducing scar tissue formation to create a stoma having a smaller diameter than surgically created waste collection reservoir 9. In some cases, stricture 7 can include artificially implanted material (e.g., a ring of material such as mesh material) configured to provide rigidity to the stricture.

Ostomy plug device 10 extends from an internal portion of surgically created waste collection reservoir 9 to outside the ostomy patient. Sealing element 12 is located within an internal portion of surgically created waste collection reservoir 9, cap element 14 is located along skin 5, and tensioning element 16 extends from sealing element 12 to cap element 14. In some cases, ostomy plug device 10 can be located within fascia 3.

In some cases, an ostomy plug device provided herein can be configured to lack fluid and gas conduits that extend from outside an ostomy patient to a sealing element located within the ostomy patient. In some cases, for example when the sealing element is inflatable, an ostomy plug device can be configured to include one or more fluid and/or gas conduits capable of being used to inflate the inflatable sealing element. For example, a tensioning element can be in the form of a conduit capable of being used to inflate an inflatable sealing element.

The materials provided herein can be used by any appropriate ostomy patient or care provider. In some cases, patients or care providers of patients with ileostomy or colostomy can use an ostomy plug device provided herein.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. An ostomy plug device (10) for sealing a stoma (7) from within an ostomy patient, wherein said ostomy plug device (10) comprises a sealing element (12), a cap element (14), and a tensioning element (16) attached to said sealing element (12), wherein said cap element (14) is configured to be located outside of the body of said patient, wherein said sealing element (12) is configured to be implanted through said stoma (7) into at least a portion of a waste collection reservoir (9) located within said patient, and wherein said tensioning element (16) is configured to provide tension to said sealing element (12),
said tensioning element (16) is attached to said cap element (14) for anchoring therefore providing tension to said sealing element (12) to seal said stoma (7), **characterized in that** the tensioning element (16) is configured to be manually pulled on one end of the tensioning element (16) to adjust the tension applied by the tensioning element (16) on the sealing element (12).

2. The ostomy plug device of claim 1, wherein said sealing element (12) is disc shaped.

3. The ostomy plug device of any one of claims 1-2, wherein:
(a) said sealing element (12) is biased to have a concave surface facing said stoma (7), wherein said tension applied to said sealing element (12) by said tensioning element (16) causes said concave surface to evert to a convex surface;
(b) said sealing element (12) is flexes upon application of said tension.

4. The ostomy plug device of any one of claims 1-3, wherein said cap element (14) is rigid.

5. The ostomy plug device of any one of claims 1-4, wherein said tensioning element (16) is a cord or string.

6. The ostomy plug device of any one of claims 1-5,wherein said tensioning element (16) is solid and lacks a lumen.

## Patentansprüche

1. Eine Ostomie-Steckvorrichtung (10) zum Verschließen eines Stomas (7) innerhalb eines Stomapatienten, wobei jene Ostomie-Steckvorrichtung (10) ein Abdichtelement (12), ein Kappenelement (14) und ein an jenem Abdichtelement (12) angebrachtes Spannelement (16) umfasst, wobei jenes Kappenelement (14) so konfiguriert ist, dass es außerhalb des Körpers jenes Patienten angeordnet ist, wobei jenes Abdichtelement (12) so konfiguriert ist, dass es durch jenes Stoma (7) in mindestens einen Teil eines Abfallsammelreservoirs (9) implantiert wird, das sich innerhalb jenes Patienten befindet, und wobei das Spannelement (16) so konfiguriert ist, dass es eine Spannung auf jenes Abdichtelement (12) ausübt,
jenes Spannelement (16) an dem Kappenelement (14) angebracht ist, um es zu verankern und daher Spannung auf das Abdichtelement (12) auszuüben, um jenes Stoma (7) abzudichten, **dadurch gekennzeichnet, dass** das Spannelement (16) so konfiguriert ist, dass es manuell an einem Ende des Spannelements (16) gezogen werden kann, um die vom Spannelement (16) auf das Dichtungselement (12) ausgeübte Spannung einzustellen.

2. Die Ostomie-Steckvorrichtung gemäß Anspruch 1, wobei das Abdichtelement (12) scheibenförmig ist.

3. Die Ostomie-Steckvorrichtung gemäß den Ansprüchen 1-2, wobei:
(a) das Abdichtelement (12) vorgespannt ist, so dass es eine konkave Oberfläche aufweist, die jenem Stoma (7) zugewandt ist, wobei die durch jenes Spannelement (16) auf jenes Abdichtelement (12) ausgeübte Spannung bewirkt, dass jene konkave Oberfläche in eine konvexe Oberfläche umkippt;
(b) jenes Abdichtelement (12) bei Anwendung der Spannung gebogen wird.

4. Die Ostomie-Steckvorrichtung gemäß den Ansprüchen 1-3, wobei jenes Kappenelement (14) starr ist.

5. Die Ostomie-Steckvorrichtung gemäß den Ansprüchen 1-4, wobei jenes Spannelement (16) eine Schnur oder ein Faden ist.

6. Die Ostomie-Steckvorrichtung gemäß den Ansprüchen 1-5, wobei jenes Spannelement (16) fest ist und kein Lumen aufweist.

## Revendications

1. Dispositif de bouchon de stomie (10) pour sceller une stomie (7) depuis l'intérieur d'un patient ayant subi une stomie, dans lequel ledit dispositif de bouchon de stomie (10) comprend un élément de scellement (12), un élément capuchon (14), et un élément de tension (16) fixé audit élément de scellement (12), dans lequel ledit élément capuchon (14) est configuré pour se trouver à l'extérieur du corps dudit patient, dans lequel ledit élément de scellement (12) est configuré pour être implanté à travers ladite stomie (7) dans au moins une partie d'un réservoir de collecte de déchets (9) situé à l'intérieur dudit patient, et dans lequel ledit élément de tension (16) est configuré pour fournir une tension audit élément de scellement (12),
ledit élément de tension (16) est fixé audit élément capuchon (14) pour l'ancrage fournissant donc une tension audit élément de scellement (12) pour sceller ladite stomie (7), **caractérisé en ce que** l'élément de tension (16) est configuré pour que le fait de manuellement tirer sur une extrémité de l'élément de tension (16) permette d'ajuster la tension appliquée par l'élément de tension (16) sur l'élément de scellement (12).

2. Dispositif de bouchon de stomie selon la revendication 1, dans lequel ledit élément de scellement (12) est en forme de disque.

3. Dispositif de bouchon de stomie selon l'une des revendications 1 et 2, dans lequel :
(a) ledit élément de scellement (12) est sollicité pour avoir une surface concave face à ladite stomie (7), dans lequel ladite tension appliquée audit élément de scellement (12) par ledit élément de tension (16) amène ladite surface concave à s'inverser en une surface convexe ;
(b) ledit élément de scellement (12) est fléchi sous l'effet de l'application de ladite tension.

4. Dispositif de bouchon de stomie selon l'une des revendications 1 à 3, dans lequel ledit élément capuchon (14) est rigide.

5. Dispositif de bouchon de stomie selon l'une des revendications 1 à 4, dans lequel ledit élément de tension (16) est une corde ou une ficelle.

6. Dispositif de bouchon de stomie selon l'une des revendications 1 à 5, dans lequel ledit élément de tension (16) est solide et n'a pas de lumière.
